Europäisches Patentamt

European Patent Office

Office européen des brevets

(11) Publication number: **0 336 800**
**A1**

## EUROPEAN PATENT APPLICATION

(21) Application number: **89400737.6**

(22) Date of filing: **16.03.89**

(51) Int. Cl.⁴: **G 01 N 33/94**
**G 01 N 33/535**

(30) Priority: **18.03.88 US 170317**

(43) Date of publication of application:
**11.10.89 Bulletin 89/41**

(84) Designated Contracting States:
**AT BE CH DE ES FR GB GR IT LI LU NL SE**

(71) Applicant: **IDETEK, INC.**
**1057 Sneath Lane**
**San Bruno California 94066 (US)**

(72) Inventor: **Singh, Prithipal**
**25627 Elena Road**
**Los Altos Hills California 94022 (US)**

**Ram, Bhanu P.**
**853-3 Greenridge**
**Daly City, California 94014 (US)**

(74) Representative: **Ahner, Francis et al**
**CABINET REGIMBEAU 26, avenue Kléber**
**F-75116 Paris (FR)**

(54) Methods and kit for determining sulfamethazine in animal fluids or feed.

(57) Methods and compositions are provided for detecting sulfa drugs, particularly sulfamethazine, in animal fluids or feed. The method employs a competitive enzyme immunoassay with bound anti-sulfamethazine sera where HRP-sulfamethazine conjugate competes with sulfamethazine in the sample for the limited amount of receptor. After removing non-specifically bound conjugate, substrate is added and the product determined. Kits can be provided for conveniently carrying out the assay.

EP 0 336 800 A1

Bundesdruckerei Berlin

## Description

## METHODS AND KIT FOR DETERMINING SULFAMETHAZINE IN ANIMAL FLUIDS OR FEED.

### INTRODUCTION

The subject field is diagnostic assays specifically for sulfonamide antibiotics in meat products.

Sulfonamide antibiotics are commonly incorporated in swine feed as promoters of growth and to control certain diseases in animals. These antibiotics are retained in tissues of animals eating a medicated diet. Consumption of meat from animals containing these antibiotic residues may result in development of hypersensitivity to these drugs and preferential selection of bacterial mutants that are resistant to these antibiotics, which antibiotics also find use in the treatment of human diseases. Normally, tissue residues in animals are controlled by withdrawing the antibiotics from feed weeks before their slaughter when concentration of the sulfonamides are presumed to reach a safe level. However, this procedure results in a number of animals reaching slaughterhouses with a substantially excessive amount of unmetabolized drugs still present in their tissue. Sulfamethazine has been identified as a major problem in approximately 95 percent of all sulfonamide tissue violations in the United States. The Food and Drug Administration now requires a 15-day withdrawal period for feed containing sulfamethazine and has established its tolerance limit in tissues for human consumption as 0.1 ppm.

Current methods for analysis of sulfamethazine in animal tissues and feeds require extensive sample extraction and cleanup and, therefore, are not readily applicable to routine screening of the drug in a large number of samples. While enzyme immunoassays have recently been reported as alternative methods for detection of sulfamethazine, these methods require extraction of the antibiotic from the sample and have long assay protocols rendering them impractical for routine analysis in abattoirs.

Relevant Literature

Fleeker and Lovett, J. Assoc. Off. Anal. Chem. printed in 1985 (68:172-174) describe an enzyme immunoassay for sulfamethazine residues in swine blood. Fleeker, Methods of Enzymatic Analysis, 3rd Ed., Vol. XII, Bergmeyer, et al., Eds., 1986, pp. 391-401 describe a procedure for detection of sulfamethazine residues in animal blood. Dixon, et al., 85th Annual Meeting Soc. Microbiol., Las Vegas, Nevada, March 3-7, 1985, describe the preparation of a monoclonal antibody for detection of sulfamethazine residues and a direct competitive enzyme-linked immunoassay. Bratton, et al., J. Biol. Chem. (1939) 28:537-550 describe a colorimetric method for detecting sulfamethazine. Manuel and Steller, J. Assoc. Off. Anal. Chem. (1981) 64:794-799 describe a gas-liquid chromatographic method of detection of sulfamethazine. Cox and Krzeminski, ibid., (1982) 65:1311-1315 describe an HPLC procedure, while Williams (Ed.), Official Methods of Analysis, (1984, Arlington, Virginia) 14th Edition, pp.785-686, by the Association of Official and Local Chemists; and Beville, et al., J. Agric. Food Chem (1978) 26:1201-1203, describe a thin layer chromatographic procedure.

A simple rapid enzyme immunoassay is provided for determining sulfamethazine in an animal-related source, such as blood, urine, tissue or feed. Enhanced sensitivity is achieved employing different linking moieties for the immunogen conjugate and the enzyme conjugate.

Methods and compositions are provided for detecting sulfamethazine from an animal fluid, e.g. red blood cell-free blood, plasma or serum, or urine, which correlates with sulfamethazine levels in animal tissue. Also, sulfamethazine in animal feed or tissue may be determined. Particularly, an enzyme immunoassay is performed using swine fluid, tissue or feed as the sulfamethazine source in a competitive assay employing antisera specific for sulfamethazine and a sulfamethazine-enzyme conjugate, particularly with horseradish peroxidase as the enzyme.

The method involves employing a limiting amount of antisera bound to a solid surface, particularly wells of a microtiter plate or test tubes. The enzyme conjugate and sample are mixed together in an appropriately-buffered medium. The resulting assay medium is added to the support-bound antisera and incubated at a moderate temperature, generally from about 20 to 40°C for at least about 5 min, more usually at least about 15 min, and not more than 2 hours, preferably not more than about 1 hour. After completion of the incubation time, the wells are washed thoroughly with an appropriate aqueous medium, e.g. an appropriately-buffered medium or tap water, usually at least about two times, more usually at least about three times, and not more than about six times, to ensure the substantially complete removal of non-specific binding of the enzyme conjugate.

To the solid substrate to which enzyme conjugate is bound in proportion to the amount of sulfamethazine present in the sample is added a non-limiting amount of substrate, which provides for a visually- or spectrophotometrically-detectable product. The medium is incubated for sufficient time for color to form; any color formation or absorbance may be detected visually or by any convenient spectrophotometric or colorimetric reader. By comparing the result with standards having known amounts of sulfamethazine, the amount of sulfamethazine may be determined. Where a visual determination is made, a standard tube having color developed with a known amount of sulfamethazine may be employed for comparison. In this way, the amount of sulfamethazine present in the sample may be correlated.

In carrying out the assay with blood, plasma or serum is obtained from blood by collecting the blood in tubes containing a metal complexing agent, e.g. EDTA, and centrifuging the sample, whereby cells are collected at the bottom of the tube and the supernatant is substantially cell-free. The supernatant is then used as the sample.

When the sulfamethazine in tissue is to be determined, the tissue is extracted as per AOAC Official Methods of Analysis (l984), sections 4l.053 to 4l.056. In the final step of the extraction procedure, the methylene chloride layer is collected, evaporated to dryness and the residue dissolved in a suitable aqueous medium, e.g. water, mixtures of water and dimethylformamide, ethanol, etc., or a polar water-miscible organic solvent, e.g. DMF, ethanol, DMSO, etc.

With feed, the feed is blended in a high-speed mixer with a water-soluble polar organic solvent or aqueous mixture thereof, e.g. l-75% DMF, and the resulting suspension filtered. The filtrate may then be used directly as the sample in the assay.

Thus, except for urine, the sample will usually be pretreated by removing red blood cells in the case of blood or by extraction with an organic solvent in the case of tissue (meat) or feed.

The antibodies are prepared by conjugation of sulfamethazine to an appropriate immunogen, particularly, the sulfamethazine is diazotized and conjugated to bovine thyroglobulin. After the conjugation, the protein is purified, in accordance with conventional ways and then used as an immunogen by appropriate immunization of a domestic animal, e.g. a rabbit. Conveniently, the mammalian host may be repetitively immunized on a schedule of from about 2 to 4 weeks between immunizations, with various adjuvants, e.g. complete and incomplete Freund's adjuvants, and the formation of antisera monitored by sampling the blood 3 days after each immunization. When it appears that the titer has approximately leveled off, the animal may be bled and the antisera isolated in accordance with conventional ways. While it has not proven necessary, the antisera may be concentrated in the desired antibodies, by employing an affinity column, having a sulfamethazine analog bonded to a solid surface. In addition, antibodies which bind strongly to sulfamethazine analogs may be removed by employing such analogs in an affinity column to preferentially remove the cross-reacting antibodies.

While the same procedure employed for linking the sulfamethazine to the immunogen may be employed for linking the sulfamethazine to the enzyme, particularly horseradish peroxidase, preferably a different method of linking is employed. Desirably, the sulfamethazine is bonded to glycosylated sites of the enzyme by cleaving enzyme sugars with a glycol cleaving reagent, such as periodate, osmium tetroxide, or the like. The resulting aldehydes may then be used for linking aliphatic diamines, which diamines may be separated by a chain of up to about 2 to 6 atoms, particularly carbon and chalcogen (oxygen and sulphur). Normally, the resulting amine is reduced to the alkylated amine by employing a borohydride under conventional conditions. The resulting aminated enzyme may now be used for conjugation to a sulfamethazine analog.

Conveniently, the amino substituent of the sulfamethazine may be functionalized to form a carbamoyl ester, employing a chloroformate ester and the resulting carbamoyl ester combined with the amino-modified enzyme to provide urea linkages. Desirably, the number of sulfamethazines per enzyme will be at least about one, more usually at least about 10, and may have up to about 65, desirably having at least about 90 percent of the available amino groups conjugated, more preferably at least about 95 percent of the available amino groups. At least about 50 percent of the enzyme activity is retained, preferably at least about 75 percent of the enzyme activity is retained as compared to the enzyme prior to conjugation of the sulfamethazine.

The antisera is non-diffusibly bound to the solid support, particularly in a manner to provide for substantially complete availablility of the binding sites of the antisera. This can be achieved by non-diffusibly binding to the surface a protein specific for immunoglobulin. Conveniently, S. aureus protein A may be employed for the binding. The binding of the antisera receptor may be achieved by incubating the surface with a buffered solution of protein at a mild temperature, preferably in the range of about 0 to 20°C for a time sufficient for the antisera receptor to become adsorbed to the surface in an irreversible manner. The surface may then be washed repetitively to remove any protein which is inadequately bound, followed by a protein wash to ensure that all "hot spots" on the surface are covered to prevent non- specific binding of proteins during the assay. After washing the surface again to remove any reversibly-bound protein, the surface is then incubated with the antisera under mild conditions, whereby the antisera becomes non-diffusibly and substantially irreversibly-bound to the surface by complexing with the antisera receptor. The surface is washed as described previously to remove any reversibly-bound antisera. By irreversible binding is intended binding which is substantially irreversible under the conditions of storage and use. The amount of antisera applied may be determined empirically to optimize the response. Conveniently, different dilutions may be applied which each antisera batch and a response curve determined.

As already indicated, depending on the sample source prior treatment may be required. Once the sample has been obtained in a form where the sulfamethazine is in a water-miscible liquid, the solution may be directly used in the assay.

The sample is combined with the enzyme conjugate in an appropriate ratio for the assay in a buffered medium. Any convenient buffer may be employed, such as tris, phosphate, etc., phosphate-buffered saline being preferred. The pH will generally vary from about 6 to 9, more usually from about 6.5 to 8. In addition, small amounts of a protein may be included, generally of about 0.005 to 0.l percent (w/v). The enzyme solution will generally be about $10^4$-$10^6$ weight-percent in enzyme, which will be combined with the sample in a ratio of about l-50:l. Usually, the sample volume will be from about 0.l to 5 ml, more usually from about 0.2 to 2 ml. Desirably,

3

the incubation temperature during the competition between the sulfamethazine in the sample and the conjugate will be above 20 C, preferably above 30°C, more preferably in the range of about 30 to 40°C, particularly 37°C.

The wash solution will be tap water, distilled water or a buffered solution, usually buffered in the range of about 6.5 to 8, preferably 7 to 7.5, having buffer in about 10 to 100 mM, preferably about 10 to 50 mM, more particularly employing as the buffer PBS. Desirably, a small amount of a non-ionic detergent will be employed, generally in the range of about 0.01 to 0.1 percent (v/v).

To the solution will then be added from about 0.1-1 volume to volume of the assay medium of a substrate solution. The substrate solution will include hydrogen peroxide or a hydrogen peroxide source and a leuco dye, which may be any one of the various substrates of horseradish peroxidase, such as 2,2'-azino(3-ethylbenzthiazoline)sulfonic acid (ABTS), ortho-chlorophenol, tetramethylbenzidine, etc. Preferably, ABTS is employed to provide the desired sensitivity.

After about 10 to 30 min, the reaction is terminated by the addition of an inhibitory solution, e.g. from about 0.5 to 2 percent of an anionic detergent, e.g. sodium dodecylsulfate, and the absorbance of the solution read. Where ABTS is employed, the reading may be conveniently in the visible region, at about 400-410 nm. By comparing the result with standards, the amount of sulfamethazine can be defined.

The results may readily be correlated with permissible levels of sulfamethazine in animal tissue or in feed. By having a series of controls with known amounts of sulfamethazine, curves can be drawn, so that the sample can be directly related to a value on the curve. Various techniques provide for linearizing the control results in a straight line, e.g. concentration vs. LOGIT % binding, so that there is an easy extrapolation and determination for each observed sample.

Kits can be provided which would include the coated microtiter well plate or test tubes, enzyme conjugate, and, as appropriate, substrate solution. Also, one or more vials of sulfamethazine, desirably in animal plasma or serum in lyophilized form may also be included. The amounts of reagent may be provided to ensure optimal use of the kit.

The following examples are offered by way of illustration and not by way of way limitation.

## Materials and Methods

### Materials.

Sulfamethazine, sodium nitrite, thimerosal, bovine thyroglobulin, Sephadex® G-50, horseradish peroxidase (HRP) (Type VI), sodium metaperiodate, sodium borohydride, casein, 2,2'-azino (3-ethylbenzthiazoline)sulfonic acid (ABTS), sodium dodecylsulfate (SDS), ammonium sulfate, 30 percent hydrogen peroxide, and 2,4,6-trinitrobenzenesulfonic acid (TNBS) were purchased from Sigma Chemical Co. (St. Louis, MO). Dimethylformamide (DMF), succinic anhydride, p-nitrophenyl chloroformate, 2,2'-oxy bis-(ethylamine) dihydrochloride were purchased from Aldrich Chemical Co. (Milwaukee, WI). Tween-20 and recombinant Protein A (r-Protein A) were bought from J.T. Baker Chemical Co. (Phillipsburg, NJ) and Behring Diagnostics (La Jolla, CA), respectively. All other inorganic and organic chemicals were of reagent or better grade. Disposable syringe filters (0.45 μm) were purchased from Glass Works (Corning, NY). Nunc microtiter plates (high binding) were from Denmark.

New Zealand female white rabbits were used for antibody production. Blood samples from trichina positive and trichina negative swine were procured from laboratory animals raised under controlled conditions. Field samples of plasma were obained from a commercial hog slaughterhouse (Lundy Packing Co., Clinton, NC).

### Preparation of Anti-Sulfamethazine Antibodies.

Fifty-seven milligrams of sulfamethazine was diazotized and conjugated to 100 mg of bovine thyroglobulin according to the published procedure (Fleeker and Lovette, 1985). The conjugate was dialyzed four times against 50 mM ammonium bicarbonate solution and purified on a column of Sephadex® G-50. Fractions containing protein were pooled, lyophilized and stored at -20°C.

### Production of Anti-Sulfamethazine Antibody.

One milligram each of the sulfamethazine-thyroglobulin conjugate in 0.5 mL saline mixed with 1 mL of complete Freund's Adjuvant was injected into each of the four New Zealand female white rabbits. After 5 weeks, each rabbit was boosted by injecting 0.5 mg of the conjugate in 0.25 mL of saline emulsified with 0.5 mL of incomplete Freund's Adjuvant. The animals were bled periodically (test bleed) through the ear vein and the serum was separated by centrifugation at 15,000 g for 20 min. Anti-sulfamethazine antibodies were purified from the serum by precipitation with ammonium sulfate. Titers of the antibodies were determined by a direct conjugate binding assay described later in this section. Rabbits were boosted every 2 weeks until a satisfactory titer was obtained.

### Preparation of Sulfamethazine-Horseradish Peroxidase (Sulfamethazine-HRP) Enzyme Conjugate.

a) Amination of HRP:

HRP (25 mg) was dissolved in 1 mL of 5 mM sodium aoetate buffer, pH 4.5 and dialyzed against the same buffer at 4°C. The HRP solution was diluted to 2 mL with the buffer and cooled in an ice-bath. Sodium metaperiodate (0.2M solution 20 µL) was added dropwise over 30 min to the stirring solution of HRP. The solution was then dialyzed three times against 2 mM sodium acetate buffer, pH 4.5. The dialyzed solution was cooled in ice and treated with 200 µL of 0.2 M 2,2'-oxy bis(ethylamine)dihydrochloride for 5 hours at 4°C with slow stirring. The reaction mixture was further incubated with 200 µL of 0.1 M sodium borohydride at 4°C overnight. The modified HRP solution was dialyzed with three changes of 0.1 M carbonate-bicarbonate buffer, pH 9.1 at 4°C. The protein content of the aminated HRP was determined by its absorbance (A) at 403 nm using an extinction coefficient of 2.275 $g^{-1}$ L $cm^{-1}$.

b) Conjugation of sulfamethazine to aminated HRP:

Sulfamethazine (36 mg, 1 mM) was dissolved in 1 mL of DMF and cooled to -15°C in an ice-salt bath. Crystals of p-nitrophenyl chloroformate (26 mg, 1 mM) were added and the reaction mixture stirred for 30 min. The aminated HRP (1 mg) was dissolved in 2 mL of 1:9 mixture of DMF and 0.1 M carbonate-bicarbonate buffer (pH 9.1) and the solution cooled in an ice-bath. The activated sulfamethazine (10 µL) prepared above was then added to the enzyme solution and the cocktail stirred at 0-4°C for 2 hours. The conjugate thus obtained was dialyzed three times against 1 L of 10 mM phosphate buffered saline (PBS), pH 7.2. Aqueous 10 percent sodium thimerosal was added to the conjugate solution to bring the effective concentration of the former to 0.01 percent. The solution was then filtered through a 0.45 µM filter disk and stored at -20°C in 50 percent glycerol.

c) Determination of amino group in HRP:

Moles of free amino groups in HRP were determined by reaction with trinitrobenzene sulfonic acid (TNBS) using a molar extinction coefficient of TNBS modified amino group of $10^4 M^{-1} cm^{-1}$ (Habeeb, Anal. Biochem. (1966) 14:323-331). Moles of sulfamethazine present in the sulfamethazine-HRP conjugate were indirectly calculated from the difference in amino groups present before and after conjugation.

Separation of Plasma from Swine Blood.

Swine blood was collected in test tubes containing EDTA (1.8 mg EDTA/mL blood) and spun at 15,000 g for 20 min at 4°C. The supernatant (plasma) was removed and stored at 4°C in the presence of 0.01 percent thimerosal.

Preparation of Sulfamethazine Standards in Plasma.

A 1 mg/mL master stock solution of sulfamethazine was prepared in DMF. The master stock solution was diluted with 20 mM PBS, pH 7.2, to give a stock solution of 100 µg/mL of the drug. The stock solution was further diluted with plasma to make a working stock of 1 µg/mL sulfamethazine. Sulfamethazine standards were prepared by making serial dilutions of 1 µg/mL working stock solution. Each standard was divided into 0.5 mL aliquots in vials and lyophilized. Lyophilized standards were stored at -20°C.

Immobilization of Antibody on Microtiter Wells.

Each well of the microtiter plate was filled with 200 µL solution of r-Protein A (2 µg/mL in 10 mM PBS, pH 7.2). These strips were sealed and incubated at 4°C for 5 hours. The r-Protein A solution was aspirated and the wells were washed 5 times with saline-Tween (0.9 percent NaCl containing 0.05 percent Tween-20). Each well was then incubated with 300µL solution of 0.1 percent casein in 20 mM PBS, pH 7.2, at 37°C for 30 min and washed as described above. Each well was then filled with 200µL of antibody solution (12.5 µg/mL in 50 mM carbonate-bicarbonate buffer, pH 9.6) and incubated at 4°C overnight (15-20 hours). The solution was aspirated and the wells air-dried at room temperature for 2-3 hours. The antibody immobilized microtiter wells thus prepared were stored in ziplock bags at 4°C.

Competitive Direct Enzyme Immunoassay (EIA).

The enzyme conjugate (sulfamethazine-HRP) was appropriately diluted (generally 1:1000) with 20 mM PBS, pH 7.2, containing 0.01 percent casein (w/v) and mixed with standards or samples in 10:1 ratio. The mixture was transferred to wells (20 L/well) and incubated at 37°C for 1 hour. The wells were washed five times with 20 mM PBS, pH 7.2, containing 0.05 percent Tween-20 (v/v). Each well was then filled with 100 µL of ABTS substrate solution (combine 1:1 (v/v) 1mM ABTS, 0.1M citrate, pH 4.0, and 0.01% thimerosal with 0.01% $H_2O_2$, and 0.01% thimerosal) and incubated at 37°C for about 20 min for color (bluish-green) development. The color development was stopped with 100 µL of 1 percent SDS solution (w/v). Absorbance of the color was monitored at 405 nm in an EIA plate reader. Percent binding was calculated from the absorbance contained in the absence (B') and presence (B) of sulfamethazine in standards/samples as follows: % binding = (B/B') x 100.

A standard curve was prepared by plotting log sulfamethazine concentration vs. percent binding. The content of sulfamethazine in an unknown sample was determined from the standard curve.

Determination of Sulfamethazine by TLC.

The test was performed in accordance with USDA handbook on Sulfa-On-Site test (SOS). The TLC plate was spotted with sulfamethazine standards (0.4 and 1.3 µg/mL) and unknown plasma samples and developed with methanol followed by ethyl acetate. Sulfamethazine in plasma samples was determined by comparing their fluorescence intensities with those of the standards.

RESULTS

Antibody Production and Evaluation.

Titer of antibodies purified from serum of each rabbit bled at various intervals were determined by a conjugate binding assay. (In this assay, appropriately diluted conjugate was added to wells coated with antibody purified with each bleed at l0 μg/mL protein concentration and the assay performed as described above under competitive direct EIA.) Each rabbit gave a slightly different response to antibody formation during the immunization period. Generally, a successive increase in titer of the antibody was observed in each case, peaking off around 60-90 days during the course of initial and three booster injections. Titers of the antibodies generally fell and fluctuated during subsequent booster injections.

In order to verify whether the color development during conjugate binding assay was due to a specific reaction involving antisulfamethazine antibodies, a competitive EIA was performed at 0 μg/mL and 0.l μg/mL of sulfamethazine. Sulfamethazine competed effectively with sulfamethazine-HRP conjugate for antibody binding. The inhibition in binding as indicated by decrease in absorbance at 0.l μg/mL relative to 0 μg/mL successively increased in spite of decrease in titer of the antibodies for rabbit No. 4. This indicated that sensitivity of the assay was independent of titer of the antibody.

Standard EIA Curve.

Since one primary objective of the study was to determine sulfamethazine in swine plasma, standards in plasma (0.0l to l μg/mL in sulfamethazine) were used to generate standard curves. A linear dose response curve was obtained by plotting log-logit of percent binding against sulfamethazine concentration.

Sulfonamides including sulfamethazine are reversibly bound to plasma proteins (Anton, J. Pharmacol. (1961) 134:291 -303). The use of standards prepared in plasma thus should balance out any interference arising due to interaction of sulfamethazine with the sample plasma proteins. Standard curves obtained by using plasma standards from three different pigs maintained on sulfamethazine-free diet were identical, indicating little or no effect by any variation in plasma from different animals. Plasma standards can be stored lyophilized at -20°C to avoid any deterioration. The dose response curves using plasma standards before and after lyophilization were essentially identical indicating no loss in the drug concentrations during lyophilization.

Recovery of Sulfamethazine.

The accuracy of the methods was checked by recovery experiments. Known amounts of sulfamethazine were added in swine plasma in varying concentration and assayed in quadruplicates. Recovery of sulfamethazine ranged from 97.6 percent to 112.5 percent with a mean value of 103.3 percent.

TABLE 1

RECOVERY OF SULFAMETHAZINE SPIKED IN
PLASMA AT VARIOUS CONCENTRATIONS BY
EIA

| Sulfamethazine (μg/mL) | | |
|---|---|---|
| Added | Recovered | % Recovery |
| 0.016 | 0.018 | 112.5 |
| 0.031 | 0.032 | 103.2 |
| 0.063 | 0.067 | 106.3 |
| 0.125 | 0.122 | 97.6 |
| 0.250 | 0.251 | 100.4 |
| 0.500 | 0.500 | 100.0 |

Sensitivity of the Assay.

A 50% inhibition in binding was obtained around 0.l μg/mL of sulfamethazine which falls in the mid-region of the EIA standard curve. The lower limit of detection of sulfamethazine was 0.0l μg/mL.

Field Study.

Fifteen field and two laboratory samples of swine plasma were analyzed by EIA and results compared with the SOS test. Both methods showed three positives with EIA values ranging from 0.35 to 0.48 μg/mL while the corresponding values by SOS method were approximately 0.4 μg/mL.

TABLE 2A

DETERMINATION OF SULFAMETHAZINE IN
FIELD PLASMA SAMPLES BY EIA AND SOS TEST

| Sample No. | Sulfamethazine (µg/mL) | |
| | EIA | SOS |
| --- | --- | --- |
| 1 | 0.35 | 0.4 |
| 2 | 0.42 | 0.4 |
| 3 | 0.48 | 0.4 |
| 4 | 0.0 | ND |
| 5 | 0.0 | ND |
| 6-17 | 0.0 | ND |

Negative for Trichinella spiralis.

Positive for Trichinella spiralis.

None detected.

Trichinosis is a common problem in swine affecting both producers and consumers. In order to test whether plasma from swine suffering from trichinosis interfered in the sulfamethazine EIA, a known trichina-positive sample was obtained and compared with a trichina-negative sample (Table 2A). Both samples were negative for sulfamethazine indicating no interference due to trichinosis in pigs.

Samples of plasma and serum were collected from hogs on a high sulfamethazine diet and from the same hogs after withdrawing the drug from the feed for a certain number of days. Levels of sulfamethazine in plasma and serum from the same animals as determined by EIA were compared with drug levels obtained by a quantitative TLC method. The following table indicates the results.

TABLE 2B

EVALUATION OF SULFAMETHAZINE INCURRED PLASMA/SERUM SAMPLES BY EIA AND TLC;
STATISTICAL ANALYSIS OF DATA

| | Plasma vs. Serum | | EIA vs. TLC | |
| | EIA | TLC | Serum | Plasma |
| --- | --- | --- | --- | --- |
| No. of Sample (n) | 180 | 23 | 23 | 23 |
| Correlation Coefficient (r) | 0.98 | 0.98 | 0.89 | 0.9 |
| Slope | 0.93 | 1.14 | 0.75 | 0.93 |

The above results show the high correlation between serum and plasma as well as between the subject EIA and the standard TLC method.

Precision of the Assay.

Precision of the EIA was analyzed by repeated determinations of carefully prepared plasma samples. Inter-well coefficient of variation (CV) of the absorbance at three different sulfamethazine concentrations ranged from 4.9 to 9.1 percent with a mean of about 7 percent.

Inter-assay CV of the percent binding of the standards on 14 different days ranged from 2.2 percent at 0.016 µg/mL to 11.4 percent at 1 µg/mL with a mean of 5.4 percent.

TABLE 3

INTER-WELL VARIABILITY OF ABSORBANCE IN
EIA OF SULFAMETHAZINE

| Sulfametha-zine[1] (µg/mL) | Absorbance | % CV |
|---|---|---|
| 0 | 1.978 ± 0.097 | 4.9 |
| 0.1 | 0.975 ± 0.063 | 6.5 |
| 0.5 | 0.395 ± 0.036 | 9.1 |

[1]Each sample was assayed in replicates of 32 in a single run.

CV, in general, increases with an increase in sulfamethazine concentration due to subsequent decrease in absorbance value.

TABLE 4

INTER-ASSAY VARIABILITY OF EIA STANDARD
CURVE FOR SULFAMETHAZINE

| Sulfameth-azine Stan-dards[1] (µg/mL) | % Binding | | % CV |
|---|---|---|---|
| | Mean | SD | |
| 0.016 | 83.1 | 1.8 | 2.2 |
| 0.031 | 76.0 | 1.8 | 2.4 |
| 0.063 | 64.2 | 2.2 | 3.4 |
| 0.125 | 48.4 | 2.5 | 5.2 |
| 0.25 | 31.4 | 1.6 | 5.1 |
| 0.5 | 18.3 | 1.5 | 8.2 |
| 1.0 | 10.5 | 1.2 | 11.4 |

[1]Each standard was assayed in quadruplicate on 14 different days

Inter-assay CV of sulfamethazine values determined from an EIA standard curve for two samples on 10 different days was less than 10 percent.

8

TABLE 5

INTER-ASSAY VARIABILITY OF
SULFAMETHAZINE IN TWO KNOWN SAMPLES
DETERMINED FROM EIA STANDARD CURVE

| Sulfameth-azine Standards[1] (µg/mL) | EIA (µg/mL) | | |
|---|---|---|---|
| | Mean | SD | % CV |
| 0.1 | 0.104 | 0.010 | 9.6 |
| 0.05 | 0.055 | 0.005 | 9.0 |

[1]Each sulfamethazine sample was assayed in quadruplicate on 10 different days. Amount of sulfamethazine in the samples was determined from the standard curve prepared on that particular day.

Low CV of the day-to-day variabilities indicated the stability of standard curve over an extended period of time. Average intra-assay CV of four samples determined on a single day was I2.8 percent.

TABLE 6

INTRA-ASSAY VARIABILITY OF
SULFAMETHAZINE EIA

| Sample No. | g/mL | | |
|---|---|---|---|
| | Mean | SD | % CV |
| 1 | 0.389 | 0.031 | 7.95 |
| 2 | 0.241 | 0.035 | 14.52 |
| 3 | 0.119 | 0.013 | 10.92 |
| 4 | 0.072 | 0.013 | 18.06 |

Each sample was assayed in replicates of 32. Individual absorbance was used to determine the amount of sulfamethazine from the standard curve.

The precision and accuracy of the sulfamethazine EIA were superior to other EIAs described for haptens.

Specificity.
Thirty (30) different sulfonamide analogues were assessed for cross-reactivity with anti-sulfamethazine antibody.

TABLE 7

CROSS-REACTIVITY OF
ANTI-SULFAMETHAZINE ANTIBODIES TOWARD
SULFAMETHAZINE ANALOGUES

| Analogues | % Cross-Reactivity[1] |
|---|---|
| Sulfamethazine | 100.0 |
| Sulfamerazine | 12.10 |
| Sulfacetamide | 0.66 |
| 5-Methoxysulfadiazine | 0.34 |
| 2-Amino-4, 6-Dimethylpyrimidine | 0.07 |
| Sulfathiazole | 0.07 |
| Sulfadimethoxine | 0.06 |
| Sulfachloropyridazine | 0.0 |
| N'-(6-Indazolyl) sulfanilamine | 0.0 |
| Sulfamethoxazole | 0.0 |
| Sulfanilyl fluoride | 0.0 |
| Sulfabenzamide | 0.0 |
| Sulfamethizole | 0.0 |
| Sulfisomidine | 0.0 |
| Sulfaguanidine | 0.0 |
| Sulfanitran | 0.0 |
| Sulfanilamide | 0.0 |
| Sulfasazine | 0.0 |
| Sulfaquinoxaline | 0.0 |
| Thymine | 0.0 |
| Sulfinpyrazone | 0.0 |
| Sulfapyridine | 0.0 |
| Sulfamethoxypyridazine | 0.0 |
| Sulfadiazine | 0.0 |
| 4-Carboxybenzenesulfonamide | 0.0 |
| N'-(4,5-Dimethyloxazol-2-yl) sulfanilamide | 0.0 |
| Sulfanilic acid, sodium salt hydrate | 0.0 |
| Sulfisoxazole | 0.0 |
| 2,6-Di-tert.-butyl-4-methyl phenol | 0.0 |
| Sulfisomidine | 0.0 |
| Sulfamide | 0.0 |

[1]Cross-reactivity defined as (nM of sulfamethazine for 50% binding/nM sulfa analogue for 50% binding) x 100.

All analogues tested, except for sulfamerazine, showed insignificant (≦1 percent) cross-reaction. It appears that epitopes for antibody recognition lie in the 2-pyrimidinyl portion of the sulfamethazine molecule. Sulfamerazine, with the only difference of one methyl group on the pyrimindinyl C-4 position, showed 12% cross-reactivity. In terms of specificity, this antibody preparation was superior to a previously reported preparation (Fleeker and Lovette, 1985, supra).

EIA for Feed.

Five grams of swine feed were blended with 50% aqueous dimethyl formamide or 0.1% NaOH (g/L) in an Osterizer blender at high speed for 5 min. After filtering the suspension through Whatman #1 paper, the filtrate was used as the sample. Standards were prepared with 50% DMF and, as required, samples were diluted with 50% DMF. The following table indicates the results.

10

TABLE 8

DETERMINATION OF SULFAMETHAZINE BY EIA
IN KNOWN SAMPLES OF FEED

| % Sulfamethazine | |
| --- | --- |
| Known Samples | EIA |
| 0.000 | $4 \times 10^{-4}$ |
| 0.011 | 0.011 |
| 1.110 | 1.2 |

On-Site EIA Test.

The test is performed using antibody-coated polystyrene tubes (l2 x 75 mm). The antibody coating procedure for the tubes is the same as described for coating microtiter wells except that r-Protein A and antibody are coated at a concentration of 5 µg/mL and l0 µg/mL, respectively with a coating volume of 0.5 mL for solution in each tube.

Using appropriate droppers, l drop ($\sim$ l0 µL) of sample standard is mixed with 5 drops ($\sim$ 500 µL) of enzyme conjugate and added to the tubes. The tubes are incubated at room temperature for l5 min after which they are washed under running tap water. Five drops ($\sim$ 500 µL) of ABTS substrate are added and the tubes incubated for l5-20 min for color development. The color in the sample tube is visually compared with the color in the control tube to see if the sample is positive or negative for sulfamethazine.

Results are evaluated as follows:

● Color in sample tube   Sulfamethazine
higher than the color in   Negative
control tube

● Color in sample tube   Sulfamethazine Positive
less than the color in
control tube

Of three samples evaluated by on-site test, one was negative and two were positive for sulfamethazine.

TABLE 9

EVALUATION OF KNOWN SAMPLES BY ON-SITE
EIA

| Known Samples ( g/mL) | On-Site EIA* ( g/mL) |
| --- | --- |
| 0 | (-) |
| 110 | (+) |
| 11,000 | (+) |

* Cutoff 5 µg/mL

Dipstick Method.

Dipsticks (8 mm x 80 mm) are prepared by attaching a suitable membrane (8 mm x 80 mm) at the end of the sticks. Anti-sulfamethazine antibodies are immobilized on the membrane.

Anti-sulfamethazine antibodies are diluted to 10 µg/mL in protein with 40 mM carbonate/bicarbonate buffer, pH 9.6. Dipsticks are placed in antibody solution so that the membrane is fully immersed in the solution for 2 hours at room temperature. Dipsticks are removed from antibody solution and washed with 0.875% sodium chloride solution. Dipsticks are now placed in blocking solution (1% casein in 10 mM PBS containing 0.01% thimerosal) for 1 hour at room temperature and then washed as described above.

Two drops ($\sim$ 20 µL) of sample/standards are mixed with 10 drops ($\sim$ 1 mL) of enzyme conjugate in a test tube (12 x 75 mm). Antibody-coated dipsticks are placed in tubes and incubated for 15 min at room temperature. The dipsticks are removed from the tubes and washed under a gentle stream of tap water. The dipsticks are now incubated with 1 mL (10 drops) of ABTS substrate solution contained in a test tube for 15-30 min for color development. The results are evaluated by employing sulfamethazine controls and comparing the color obtained with a sample and the color obtained with a control at a predetermined cut-off concentration.

The above data show that a simple, rapid protocol is provided for the detection of sulfamethazine in animal

EP 0 336 800 A1

fluids or feed. The method does not require complicated equipment and can be readily carried out without significant skills in a reproducible and efficient manner. In addition, the reagents are relatively simple, easily stored, and stable for long periods of time. The method is highly reproducible and sensitive over the range of interest. Thus, the subject method can be used in the environment of an abbatoir where large numbers of samples can be rapidly processed in an accurate and reproducible manner.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

## Claims

1. A method for determining sulfamethazine in a naturally occurring sample, said sample comprising a bodily fluid, tissue or feed, said method comprising:

combining a bodily fluid comprising red cell free-blood or urine or an organic solvent extract of sulfamethazine containing tissue or feed with a limiting amount of support bound anti-sulfamethazine antisera and a sulfamethazine-horseradish peroxidase conjugate for a time sufficient for said conjugate and any sulfamethazine in said sample to compete for binding to said antisera, wherein said antisera is prepared from an immunogen-sulfamethazine compound having a different mode of attachment of said sulfamethazine from the mode of attachment of said conjugate, and said antisera is bound to said support by binding to a receptor for the antisera;

washing said support free of non-specifically bound conjugate;

adding substrate for said horseradish peroxidase, wherein said substrate produces a detectable signal; and

comparing the signal observed with said sample with a signal obtained with a known amount of sulfamethazine control assayed under substantially the same conditions as said sample.

2. A method according to Claim 1, wherein said support is a microtiter plate well or test tube having an initial coat of non-diffusibly bound S. aureus protein A.

3. A method according to Claim 2, wherein said substrate is ABTS.

4. A method according to Claim 1, wherein said sulfamethazine joined to said immunogen is through a diazo linkage and said sulfamethazine joined to said horseradish peroxidase is through a urea linkage.

5. A method according to Claim 1, wherein said sample is plasma or serum.

6. A method for determining sulfamethazine in swine plasma or serum, said method comprising:

combining said plasma or serum with a limiting amount of support bound anti-sulfamethazine antisera and a sulfamethazine-horseradish peroxidase enzyme conjugate for a time sufficient for said conjugate and any sulfamethazine in said sample to compete for binding to said antisera, wherein said antisera is prepared from an immunogen-sulfamethazine compound linked through a diazo group and said conjugate has sulfamethazine linked to said horseradish peroxidase through a urea group, and said antisera is bound to said support by binding to S. aureus protein A non-diffusibly bound to said support;

washing said support free of non-specifically bound conjugate;

adding ABTS as substrate for said horseradish peroxidase, wherein said substrate produces a detectable signal; and

comparing the signal observed with said sample with a signal obtained with a known amount of sulfamethazine control assayed under substantially the same conditions as said sample.

7. A method according to Claim 6, wherein said control sample is prepared with swine plasma or serum.

8. A method according to Claim 6, wherein said support is a microtiter plate well or test tube.

9. A method according to Claim 6, wherein said conjugate is prepared by glycol cleavage of sugars present on said enzyme to produce an aldehyde product, reductive amination with diamines of said aldehyde product to produce an amine modified enzyme, formation of a carbamate of sulfamethazine and conjugation of said carbamate with said amine modified enzyme to produce a sulfamethazine-enzyme conjugate wherein at least 90% of the available amino groups are reacted.

10. A method for determining sulfamethazine in a naturally occurring sample, said sample comprising a swine feed, said method comprising:

combining an organic solvent extract of sulfamethazine containing feed with a limiting amount of support bound anti-sulfamethazine antisera and a sulfamethazine-horseradish peroxidase conjugate for a time sufficient for said conjugate and any sulfamethazine in said sample to compete for binding to said antisera, wherein said antisera is prepared from an immunogen-sulfamethazine compound having a different mode of attachment of said sulfamethazine from the mode of attachment of said conjugate, and said antisera is bound to said support by binding to a receptor for the antisera;

washing said support free of non-specifically bound conjugate;

adding substrate for said horseradish peroxidase, wherein said substrate produces a detectable signal;

and
comparing the signal observed with said sample with a signal obtained with a known amount of sulfamethazine control assayed under substantially the same conditions as said sample.

11. A kit comprising a support coated with antisera to sulfamethazine, a sulfamethazine-horseradish peroxidase enzyme conjugate, wherein said conjugate is prepared by glycol cleavage of sugars present on said enzyme to produce an aldehyde product, reductive amination with diamines of said aldehyde product to produce an amine modified enzyme, formation of a carbamate of sulfamethazine and conjugation of said carbamate with said amine modified enzyme to produce a sulfamethazine-enzyme conjugate wherein at least 90% of the available amino groups of said amine modified enzyme are reacted, and sulfamethazine control compositions.

12. A kit according to Claim II, wherein said controls contain sulfamethazine in lyophilized swine serum or plasma and said support is a microtiter well, dipstick or test tube.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | JOURNAL OF THE ASSOCIATION OF OFFICIAL ANALYTICAL CHEMISTS, vol. 68, 1985, pages 172-174, US; J.R. FLEEKER et al.: "Enzyme immunoassay for screening sulfamethazine residues in swine blood" * Whole article * | 1,6,7, 10-12 | G 01 N   33/94 G 01 N   33/535 |
| Y,D | "Methods of enzymatic analysis", "Drugs and Pesticides", 3rd edition, vol. XII, 1986, pages 391-401, H.U. Bergmeyer et al., VCH, US; J.R. FLEEKER: "Sulphamethazine residues in animal blood" * Page 392, line 23 - page 400, line 32 * | 1-12 | |
| Y | CLINICA CHIMICA ACTA, vol. 81, 1977, pages 1-40, Elsevier/North-Holland Biomedical Press; A.H.W.M. SCHUURS et al.: "Enzyme-immunoassay" * Page 4, line 15; page 5, line 9 - page 8, line 34 * | 1-12 | TECHNICAL FIELDS SEARCHED (Int. Cl.4) |
| X,P | CHEMICAL ABSTRACTS, vol. 110, 1989, page 423, abstract no. 22389a, Columbus, Ohio, US; D.E. DIXON-HOLLAND et al.: "Competitive direct enzyme-linked immunosorbent assay for detection of sulfamethazine residues in swine urine and muscle tissue", & J. - ASSOC. OFF. ANAL. CHEM. 1988, 71(6), 1137-40 * Abstract * | 1-12 | G 01 N |

--- -/-

The present search report has been drawn up for all claims

| Place of search | Date of completion of the search | Examiner |
|---|---|---|
| THE HAGUE | 13-07-1989 | VAN BOHEMEN C.G. |

EPO FORM 1503 03.82 (P0401)

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,P | CHEMICAL ABSTRACTS, vol. 110, 1989, page 549, abstract no. 56050y, Columbus, Ohio, US; P. SINGH et al.: "Enzyme immunoassay for screening of sulfamethazine in swine", & J. AGRIC. FOOD CHEM. 1989, 37(1), 109-14 * Abstract * | 1-12 | |
| X | EP-A-0 016 552 (NORTHWESTERN UNIVERSITY) * Page 17, lines 1-20 * | 2 | |

TECHNICAL FIELDS
SEARCHED (Int. Cl.4)

The present search report has been drawn up for all claims

| Place of search | Date of completion of the search | Examiner |
|---|---|---|
| THE HAGUE | 13-07-1989 | VAN BOHEMEN C.G. |